# EUROPEAN PATENT APPLICATION

(11) **EP 0 663 444 A1**
(43) Date of publication of application: **19.07.1995**
(21) Application number: 94400104.9
(22) Date of filing: 17.01.1994
(51) Int. Cl.: C12N 15/10, C12N 5/06, C07K 14/47

(54) **Cell lines, maturation factors and use of said cell lines and factors**

(71) Applicant: INSTITUT NATIONAL DE LA SANTE ET DE LA RECHERCHE MEDICALE (INSERM), 75013 Paris (FR)
(72) Inventor: Ceredig, Rhodri, F-67870 Bischoffsheim (FR); Fisher, Amanda, OXON OX12 9UQ (GB)
(74) Representative: Warcoin, Jacques

(57) **Abstract**

The invention deals with bipotent immature mammalian haemopoietic precursor, pure stem cell culture, having the marker constellation AA4.1⁺B220⁻.

## Description

The invention deals with new cell lines and their use in the biological fields.

More specifically, the present invention deals with putative bipotent stem cells capable of both macrophage and B lineage differentiation which are present, for example, in the embryonic liver of mammal and have the marker constellation AA4.1⁺B220⁻. These cells are extremely rare and have been identified largely indirectly, either in retroviral marking experiments or using *in vitro* functional assays which detect their differentiated progeny.

The present invention describes the isolation and characterisation of a genetically engineered bone marrow stromal cell line which permits the outgrouwth of these normally infrequent cells.

The invention also provides cell line having the marker AA4.⁺B220⁻ and process for their selection and culture.

The stromal cell line also provides a means to facilitate the phenotypic and molecular appraisal of AA4.1⁺B220⁻ bipotent stem cells and to investigate events controlling their conversion into B220⁺ pre-B cells.

The present invention deals with bipotent immature mammalian haematopoïetic precursor, pure stem cell culture, having the marker constellation AA4.1⁺B220⁻.

Such a culture has never been obtained before although the existence of such cells *in vivo* has been suspected.

Current understanding of B lymphocyte development has been considerably advanced by the provision of stromal cell lines which in combination with exogenously added growth factors such as IL-7, allow the expansion and differentiation of bone marrow pre-B cells. Such model systems have been widely used to explore the transition of B220⁺ (B-cell committed) precursors (from bone marrow or foetal liver) through stromal- and IL-7- dependent stages, into mitogen responsive and/or immunoglobulin secreting progeny. Relatively little is yet known about earlier stages in lymphoïd development, although the recent identification of a subset of stem cells (AA4.1⁺B220⁻) with potential for both macrophage and B cell differentiation suggests that B lineage committment (at least during embryonic development may occur coincident with or just prior to the onsel of B220 expression by AA4.1⁺ cells.

The present invention also deals with cell line able to propagate stem cells culture having the marker constellation AA4.1⁺B220⁻.

Such a stromal cell line supports the expansion of early precursor cells capable of generating both B lymphocytes and macrophages. This line may be derived from the bone marrow of a transgenic mouse expressing the IL-7 gene under the control of an MHC class II promoter.

Such cell line may be immortalized by using virus like retrovirus, among said cell line B16-14 is preferred.

As will be described hereinafter, the pure cell cultures having the marker AA4.1⁺B220 are obtainable by isolation from embryonic liver cells of mammal cultured on B16-14 monolayer and sorted for expression of AA4.1 and lack of B220.

But is is also possible obtained said cell line by isolation from tumours cells of a transgenic mammal carrying IL-7 gene under the control of MHC class II promotor cultured on B16-14 monolayer and sorted for expression of AA4.1 and lack of B220.

The present invention also deals with Maturation Inhibiting Factors which suppress maturation of stem cell line AA4.1⁺B220⁻ to pro/pre B-cells which are obtainable from cell line according to the invention.

Said factor is obtainable by the separation of protein from the supernatant of B16-14 and immunologically extracting the known inhibitor and promotor from said proteins to select the Maturation Inhibiting Factor from the remaining proteins.

The technology of immunopurification is known and the monoclonal or polyclonal antibodies corresponding to know inhibiting or promoting factors are available or may be prepared in a known way.

The present invention also concerns the Cultivation Supporting Factor which support culture of stem cell line AA4.1⁺B220 which are obtainable from cell line according to the invention.

Differentiation of inhibiting and supporting factors is done directly by checking their activity on the culture all itself.

Cell lines and factors are usefull in biological field as it will be examplified hereinafter, especially for *in vitro* tests but also for *in vivo* tests and treatments.

The factors may be used for culture purpose on medium withouth supporting cells but also for treating conditions where they are usefull.

The cells may be used as host for transformation before differentiation and reimplanted in the mammal for transformation in both macrophages and lymphocytes B.

The invention also deals with the cDNA bank corresponding to the described cells both for the promotor and other regulating sequences and for the DNA coding for factors described hereinbefore.

Although no demonstration exist the factor and the cell may be interesting in pharmaceutical treatment including gene therapy.

Cell line B16-14 has been filed in European Collection of Animal Cell Cultures (ECACC) - PHLS Centre for Applied Microbiology and Research, PORTON DOWN, Salisbury, Wiltshire SP4 0JG, United Kingdom under N° 94011317.

### EXAMPLE 1

### MATERIALS AND METHODS

### Transgenic mice, cell lines and cell culture

IL-7 transgenic mice were generated using the plasmid pDOI5-IL7. This construct was designed to express the IL-7 gene under the control of the Eα promotor, by inserting the cDNA for murine IL-7 on either side of the rabbit β-globin intron of vector pDOI-5. Using conventional technology, a high copy number (C57BL/6 x DBA2)F1 male founder was derived which was backcrossed to C57BL/6 to establish the IL-7 transgenic mouse colony.

Primary adherent cultures of bone marrow cells from a transgenic mouse were established in IMDM, 10 % FCS gentamycin (100 µg/ml) and infected with the recombinant retrovirus ZipSV40tsA58 encoding a temperature sensitive version of the SV40T antigen as described. Cultures were maintained at the permissive temperature of 33°C, and G418 (500 µg/ml) was added 48 hours after infection. A single G418-resistant colony was picked and recloned. Early passages of this cell line, B16-14, were used as feeder cells, either after pretreatment with mitymycin C (25 µg/ml for 45 min. at 37°C) or growth arrest induced by culture at the non-permissive temperature of 37°C.

Liver tissue from normal 12-day C57BL/6 embryos was passed through a 25 gauge needle to yield a single cell suspension, separated over Ficoll HYpaque, and added to adherent monolayers of B16-14 cells. After 4-5 days in culture at 37°C in IMDM containing 10 % FCS (batch tested for support of CFU-B), cells remaining in suspension or loosely attached to the stromal cells were harvested and analysed. Where indicated, subconfluent cultures of B16-14 (maintained at 33°C) were incubated overnight with 200 U/ml recombinant γIFN (Genzyme). The cells were then cultured for 4-5 days at 37°C before being harvested by mild trypsinisation or EDTA treatment. Uninduced control cultures were established in parallel.

### Analysis of IL-7 transgene and SCF (c-kit ligand) mRNA expression by PCR

RNA was prepared from B16-14 cells as previously described and cDNA was snthesised using 1 µg RNA and 5U AMV RT. For SCF detection, three primers were used to allow recognition of mRNAs corresponding to soluble and membrane bound forms of SCF;
- primer 1 :: 5' CCGGATCCTGGAGCTCCAGAACAGCTAA 3'
- primer 2 :: 5' GGCTGCAGTTATTGCAACAGGGGGTAACATAAAT 3'
- primer 3 :: 5' GGCTGCAGTCCAACAATTACACCTCTTGAA 3'
PCR amplification was performed in a Perkin Elmer Cetus DNA thermal cycler over 35 cycles of 94°C for 30 min 65°C for 40 min and 72°C for 80 min The detection of transgene-derived IL-7 transcripts was performed by PCR using the following pair of primers :
- primer 4 :: 5' CGTGGATCCTGAGAACTTCAGGCTC 3'
- primer 5 :: 5' ATTCTTTTTCTGTTCCTTTAC 3'
Primer 4 identifies sequences in the splice junction of the rabbit β-globin gene segments (473-493 and 1067-1071), corresponding to nucleotides -3 to +18 juxtaposed to +575 to 578 in pDOI5-IL-7. Primer 5 corresponds to nucleotides 2345 to 2365 in the IL-7 cDNA such that a 490 bp PCR fragment is predicted from their combined use. Since primer 4 only recognises spliced transgene-derived transcripts, this strategy eliminates the potential problem of distinguishing *bona fide* RNA from DNA contaminants. The PCR conditions used were 35 cycles of 94°C for 30 min, 45°C for 40 min and 72°C for 80 min. PCR products were subjected to electrophoresis in 1,3 % agarose gels, blotted and hybridised to a ³²P labelled IL-7 specific oligonucleotide (positions 2125 to 2153 :
- primer 6 :: 5' ATGACAGGAACTGATAGTAATTGCCCG 3'

### Antibodies, immunofluorescence staining and FACS analysis

Direct immunofluorescence analysis was performed using PE-coupled anti-B220, FITC-coupled andi-CD43 (Pharmingen) and FITC-coupled anti-IgM. Biotinylated AA4.1 antibody was revealed by streptavidin-FITC. Rat monoclonal antibodies to AA4.1, CD44 (IM7 and KM81), FcR (24G2). MAC-1 (MI/70), CD3 (KT3), MHC class II (ATCC Tib120) and CD45-common determinant (MI/93) were detected with FITC-labelled sheep anti-rat Ig (Silenius) or a F(ab)₂ fragment of mouse anti-rat IgG (Jackson). Mouse monoclonal antibodies PAb 412 (anti-SV40 large T) and BP-1 (anti pre-B cell associated antigen) were visualised with FITC-labelled F(ab)₂ fragment of Fc-specific goat anti-mouse IgG (Jackson). Goat anti-vimentin antibody (ICN biomedicals, UK) was visualised using FITC conjugated swine anti-goat Ig (Tgo, USA). Staining of cells in suspension followed standard protocols and approximately 2x10⁴ events were acquired on a FACScan (Becton Dickinson). Monolayer cultures were stained following culture (either at 33°C or at 37°C) on chamberslides (Gibco) and fixation in acetone/methanol as described

### Sorting of AA4.1⁺B220⁻ and culture on ST2 monolayers

Day 12 embryonic livers celles were cultured for 5 days on B16-14 monolayers, harvested by gentle pipetting, and simultaneously stained for B220 and AA4.1 expression as described above. Usiong an Odam ATCC 3000 tow-laser flow cytomer (Odam, Wissembourg, France) cells were sorted for expresion of AA4.1 but lack B220. The phenotype of sorted cells was verified by restaining and analysis on a FACScan. Between 2x10⁴ and 1,4x10⁵ purified AA4.1⁺B220⁻ cells were obtained from approximately 1 to 3x10⁶ cultured foetal liver cells in each of seven independant experiments. Sorted cells were plated at low density on mitomycin C pre-treated ST2 monolayers. Colonies derived from single cells were identified (by marking the underside of Petri dishes) on monitored. Cultures were incubated for 7 days in the presence or absence of 2x10³ units/ml of recombinant murine IL-7. Where indicated, LPS (25 µg/ml) was added for a further 3 to 5 days. In some experiments, ST2 or B16-14 monolayers were overlayed with AA4.1⁺B220⁻ cells in 0,3 % agar. Ficoll Hypaque purified normal mouse bone marrow cells served as control progenitor populations.

### EXEMPLE 2

### CELL LINE B16-14

The stromal cell line B16-14 was derived by infection of a primary culture of IL-7 transgenic bone marrow with a recombinant retrovirus encoding a temperature-sensitive variant of the simian virus 40 T gene (SV40tsA58). The rationale for this approach was that immortilisation with a conditional oncogene would allow cells to proliferate at the permissive temperature (33°C) while a more "normal" differentiated phenotype was expected at 37-39°C (at which temperature the SV40 T is non-functional.

B16-14 stromal cells grew will at 33 °C and expressed high levels of nuclear SV40T. These cultures expressed the adhesion molecule CD44 but were unreactive with a large panel of antibodies including reagents directed to BP-1, cytokeratins, monocytes/macrophages or dendritic cells. At 37°C, B16-14 cultures stopped proliferating, expressed enhanced levels of vimentin and adopted an elongated appearence with long processes more reminiscent of fibroblastoïd cells. These cultures remained similarly unreactive with a variety of lineage associated markers, and lost detectable expression of nuclear SB40T.

### EXAMPLE 3

### CELL CULTURE AA4.1⁺B220⁻

The capacity of B16-14 to support lymphoïd development was tested using normal 12-day embryonic lever cells as a source of precursors. Freshly isolated liver cells were heterogenous containing approximately 16 % CD45-reactive cells, and a few (<4 %) AA4.1 + cells. After 4 days of co-culture with B16-14, a sligth expansion of the liver derived cells was consistantly noted (on average 1,9-fold) and two distinct populations could be clearly discerned on the basis of forward and side scatter parameters. A subset of larger cells (population II) resembled macrophages both in terms of cytochemical staining (not shown), binding of antibodies to mouse Fc receptors and Ig in normal rat serum, and reactivity with mAB to CD45 and the macrophage associated antigen MCA-1 (CDIIb). A subset of smaller cells had light scatter parameters characteristic of lymphocytes. These cells expressed CD44 and were largely unreactive with the monoclonal antibody BP-1 (13,5 % BP-I^{dim} ,not shown) interestingly, 98 % of this population expressed AA4.1 but only a small proportion (12 %) was labelled with antibody of the B-cell associated molecule B220 (specific for the CD45RA isoform). Two colour staining indicated that among the B220 positive subset most cells were pre-B (lacking surface IgM) and only 2 % displayed a more mature B cell phenotype (sIgM⁺B220⁺ lower panels). Thus B16-14 appears to support a population of immature AA4.1⁺B220⁻ foetal liver cells. These cells are phenotypically identical to recently identified stem cell subset capable of generating both macrophages and B cells *in vitro* and *in vivo*.

To investigate the lineage potential of AA4.1⁺B220⁻ cells generated in B16-14 cocultures, this population is purified by fluorescent activated cell sorting and plated the resulting cells on monolayers of ST2. This stromal line allows the generation of mitogen responsive and/or Ig⁺ progeny from B cell precursors, provided that IL-7 is supplied. By plating AA4.1⁺B220⁻ cells at low or high densities we were able to both monitor the outgrowth of colonies from single cells and to obtain sufficient cell numbers for flow cytometric analysis. In the absence of exogenously added IL-7, AA4.1⁺B220⁻ cells plated on ST2 gave rise predominantly to macrophage progeny. In the presence of IL-7 and ST2, colonies both macrophages and small lymphoïde cells were evident after 5-7 days. Analysis of bulk cultures established in parallel showed a predominant population of lymphoïd cells expressing B220 (94 % of cells) at intermediate and high levels middle profile). Four percent of these cells expressed surface IgM a proportion that increased to 17 % after a further 3 days culture in LPS. A corresponding increase both in cell size and level of B220 expression (96 % B220 bright) suggests that LPS induced the maturation of this pre-B cell population. These data confirm the potential of AA4.1⁺B220⁻ cells to give rise to both B lymphocytes and macrophages, and underscore the capacity of B16-14 cells to maintain dual-potent precursors. In additional experiments in which foetal liver or normal bone marrow cells were overlayed (0,3 % agar) on B16-14 stroma, macrophage colonies and some large colonies (> 200 cells) of mixed macrophage/blast cells were observed (data not shown). This blast cell population avaits further characterisation. However, B16-14 did not potentiate the further development of surface IgM⁺ or mitogen responsive cells from these cells (or purified AA4.1⁺B220⁻ populations), in agar overlay cultures or direct (contact) co-cultures, even after prolonged exposure to LPS. Furthermore no indication of granulocyte lineage differentiation was seen. Thus, while B16-14 supports the generation of bipotent (B/macrophage) progenitors, this line (in contrast to others such as S17 and ST2 is restricted in its capacity to promote later stages of B cell development. From the supernatant of B16-14 it is possible to that the maturation inhibiting factors and cultivation supporting factors.

### EXAMPLE 4

### CHARACTERISATION OF B16-14

The expression of IL-7 by B16-14 cells was investigated by PCR. As the IL-7 transgene carried by these cells is driven by the MHC class II Eα promoter, we sought to determine whether IL-7 expression could be enhanced by agents capable of inducing class II expression, such as γIFN. Therefore, RNA expression by uninduced and γIFN-treated cultures was assessed in parallel, B16-14 did not constitutively produce detectable IL-7 although trangene derived IL-7 was found following γIFN treatment, consistent with enhanced cell surface expresson of MHC class II. Nevertheless, uninduced B16-14 cells were able to support the survival of the IL-7 dependent cell line 2E8 upon co-culture, although under these conditions proliferation of this pre-B cell line was minimal (data not shown). Furthermore, we have observed that B16-14 cells allow the expansion of AA4.1⁺B220⁻ cells isolated from tumours arising in IL-7 transgenic mice (see example 3). Cells from these tumours proliferate in response to IL-7 in a dose dependent manner and appear critically dependent on IL-7 for their short term survival since culture in the absence of IL-7 causes rapid DNA fragmentation. That B16-14 cells can support both AA4.1⁺B220⁻ normal cells and IL-7 dependent cell lines in the absence of detectable constitutive expression of IL-7 could have a number of different explanations. Firstly, low but biologically significant levels of endogenous (not transgene derived) IL-7 may be produced by B16-14 cells. Secondly, interaction between B16-14 stromal cells and haemopoïetic precursors may stimulate IL-7 synthesis. Finally, it is possible that additional factors are elaborated by B16-14 which act (perhaps in concert with IL-7) to support the outgrowth of AA4.1⁺B220⁻ cells from foetal liver. In this context SCF is an obvious candidate, although it is perhaps worth noting that additional ligands have been implicated in these processes.

SCF (c-kit ligand) is known to act synergistically with IL-7 in promoting CFU-B and is reported to be crucial for the survival of primitive B220⁻ (Sca-1⁺Lin⁻) precursors. Although this data has been refuted by a subsequent investigation. There is good evidence that SCF can synergise with the mesenchyme derived growth factor IL-11 to promote even earlier pre-CFCₘᵤₗₜᵢ cells. Furthermore, reports of IL-7/SCF synergy have been limited to adult (bone marrow derived) lymphoid precursors which may have different properties from their embryonic counterparts (such as, for example, BP-1 expression). Therefore, we wished to determine whether B16-14 cells produce SCF. Both soluble and transmembrane forms of SCF have been identified, and at least two different mRNAs (thought to arise by alternate splicing) specify the latter (KL-M1 and KL-M2. To detect SCF expression by B16-14, a previously reported PCR strategy was used in which amplification is achieved using pairs of primers corresponding to sequences in the extracellular and/or carboxy (cytoplasmic) domains of SCF. Using a primer sct capable of recognising both soluble and KL-M1 forms of SCF (primers 1+2), a single 637bp fragment was observed in uninduced and γIFN induced B16-14 samples. The expression of KL-M1 was confirmed using primers 1 and 3 (which can detect both KL-M1 and KL-M2) as indicated by a unique band of 900bp. Thus B16-14 constituitively express the larger (KL-M1), transmembrane form of SCF, consistent with the reported tissue-specific expression of KL-M1 in bone marrow and brain tissue. These data show that B16-14 cells constitutively express SCF, allowing for the possibly that this factor has a role in the maintenance of AA4.1⁺B220⁻ precursors.

In conclusion, date presented here provide evidence that B16-14 cells support early lymphoïd/macrophage precursors, particularly those expressing AA4.1 but lacking B220. B16-14 cells constitutively express SCF and can be induced to express IL-7, consistent with the reported role of these factors in early B-cell and mycloid development. Furthermore, B16-14 cells express CD44 (Pgp1), (including the HA-binding epitope) which is thought to be a critical component in the cell recognition/adhesion events necessary for lymphohaemopoiesis (antibodies to CD44 block B-lymphopoiessis in longterm Whitlock-Witte cultures. A numbeer of bone marrow stromal cell lines have previously been reported including S17, S10, FLS4.1, BSM1, BSM2, AC4, BHM and ST2, PA6. Of these, B16-14 most closely resembles PA6 with respect to (i) affecting cells in the bone marrow or foetal liver that lack B220 expression, (ii) being unable to support the further maturation of B220⁺ cells into mitogen responsive/sIgM⁺ cells (iii) that lymphopoietic support is mediated by direct cell contact. However, the cell line PA6 is defective in IL-7 production and, in the absence of exogenously added factors, generates predominantly myeloid progeny. Although PA6 is believed to be capable of maintaining the earliest B cell progenitors in culture (as assayed by transfer of cultures to ST2 monolayers the precise phenotype of these cells has not been reported (most likely because phenotype analysis of this very minor population of cells is not feasible). The stromal line B16-14 described here provides access to sufficient numbers of functionally bipotent AA4.1⁺B220⁻ cells to allow their detailed characterisation and manipulation *in vitro*.

### EXAMPLE 5

Of 169 IL-7 transgenic mice obtained as in example 1 derived from a single founder approximately 25 % of mice developed tumours, in some cases as early as 4 weeks of oage. Among non-transgenic animals the frequency of comparable lesions was extremly low (< 0,5 %). A detailed phenotype analysis of cells derived from tumour tissues showhs that in the majority of cases (9 of 15 studied) the tumour comprises an expanded pre-B cell populaton with the predominant phenotype B220⁺AA4.1⁺ sIgM^{-.} Three samples contained cells with a less mature phenotype (B220 or low AA4.1⁺ BP-1⁺) and two tumours represented cells which were slightly more mature (MHC class II⁺ sIgM^{low}). In a single case (10-11-66) the tumour consisted of cells expressing Sca-1 and CD45 but devoid of lineage associated markers (B220, MAC-1, CD8, BP-1, AA4.1,Thy-γ ^{low}). These data suggest that the lymphoproliferative disorders apparent in IL-7 transgenic represent a spectrum of stages in B-cell development including cells which on the basis of phenotypic analysis resemble bi- or multipotent precursors.

That this tumour represented an oligoclonal population of cells was confirmed by analysing the IgH status of biologically cloned cells derived from the original tumour sample ; seven clones derived from 10.11.6 were propagated using the bone marrow cell line B16-14 (immortalised using SV40ts retrovirus) and at least three different rearrangement pattern were detected.

Isolation of B220⁻AA4.1⁺ give rise to similar result as previously described.

## Claims

1. Bipotent immature mammalian haemopoietic precursor, pure stem cell culture, having the marker constellation AA4.1⁺B220⁻.

2. Cell culture according to claim 1 obtainable by isolation from embryonic liver cells of mammal cultured on B16-14 monolayer and sorted for expression of AA4.1 and lack of B220.

3. Cell culture according to claim 1 obtainable by isolation from tumour cells of a transgenic mammal carrying IL-7 gene under the control of MHC class II promotor cultured on B16-14 monolayer and sorted for expression of AA4.1 and lack of B220.

4. Cell line able to propagate stromal cell culture having the marker constellation AA4.1⁺B220⁻.

5. Cell line according to claim 4 obtainable by culture of a stromal cell derived from bone marrow of a transgenic mammal carrying the IL-7 gene under the control of a MHC class II promotor.

6. Cell line according to claim 5 obtainable by immortalization of the stromal cells.

7. Cell line according to claim 6 wherein immortalization is done by using a retrovirus.

8. Cell line according to claim 4 which is B16-14.

9. Maturation Inhibiting Factors which suppress maturation of stromal cell culture AA4.1⁺B220⁻ to pre/pro B-cell which are obtainable from cell line according to claims 4 to 8.

10. Maturation inhibiting factors according to claim 9 obtainable by separation of proteins from the supernatant of B16-14 and immunologically extracting the known inhibitor and promotors from said proteins to select the Maturation inhibiting factors from the remaining proteins.

11. Cultivation supporting factor which support culture of stromal cell line AA4.1⁺B220⁻ which are obtainable from cell line according to claims 4 to 8.

12. Use of cell line according to claims 1 to 8 and factor of claims 9 to 11 in biological field.

13. use according to claim 12 for *in vitro* test.

14. Use according to claim 12 fo*r in vivo* test.

15. cDNA bank obtained from cell AA4.1⁺B220⁻.

16. DNA contained in the bank of claim 15 which code for promotor efficient in said AA4.1⁺B220⁻ cells.

17. cDNA bank obtained from cell according to claim 4 to 8.

18. DNA contained in the bank of claim 17 which codes for factors according to claims 9 to 11.
